# EUROPÄISCHE PATENTANMELDUNG

(11) **EP 2 371 404 A2**
(43) Veröffentlichungstag der Anmeldung: **05.10.2011**
(21) Anmeldenummer: 11156795.4
(22) Anmeldetag: 03.03.2011
(51) Int. Cl.: A61L 31/16

(54) **Medizinisches Implantat mit einer Beschichtung bestehend aus oder enthaltend mindestens einen Nitro-Statin-Wirkstoff**

(30) Priorität: 30.03.2010 US 318806 P
(71) Anmelder: Biotronik AG, 8180 Bülach (CH)
(72) Erfinder: Gratz, Matthias, 91054 Erlangen (DE); Borck, Alexander, 91086 Aurachtal (DE); Köck, Kathleen, 18581 Putbus (DE)
(74) Vertreter: Lindner-Vogt, Karin L.

(57) **Zusammenfassung**

Die Erfindung betrifft ein medizinisches Implantat, dessen Oberfläche ganz oder in Teilen mit einer Beschichtung, die aus mindestens einem Wirkstoff besteht oder mindestens einen Wirkstoff enthält, überzogen ist.

## Beschreibung

Die Erfindung betrifft ein medizinisches Implantat, dessen Oberfläche ganz oder in Teilen mit einer Beschichtung, die aus mindestens einem Wirkstoff besteht oder mindestens einen Wirkstoff enthält, überzogen ist.

Implantate haben in vielfältiger Ausführungsform Anwendung in der modernen Medizintechnik gefunden. Sie dienen beispielsweise der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, z.B. als Nagel, Platte oder Schraube.

Das Implantat besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßer Verwendung mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Eine biologische Reaktion auf polymere, keramische oder metallische Implantatwerkstoffe hängt von der Konzentration, Einwirkdauer und Art der Zuführung ab. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem der Stentimplantation in Blutgefäße ist die In-Stent Restenose. Aufgrund des intravaskulären Eingriffs kann es zu einer erhöhten Thrombusbildung sowie einer erhöhten Proliferation von glatten Muskelzellen kommen, was zu einem erneuten Gefäßverschluss (Restenose) führen kann. Überschießende Proliferation von Narbengewebe führt bei ca. 30 - 40% aller unbeschichteten Stents nach längerer Zeit zu einer Restenose.

Ein aussichtsreicher Ansatz zur Lösung des Problems liegt in der Verwendung biokorrodierbarer Metalle und deren Legierungen als Implantatwerkstoff, denn zumeist ist eine dauerhafte Stützfunktion durch den Stent nicht erforderlich; das zunächst geschädigte Körpergewebe regeneriert. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Kalzium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest <1% bekannt, die sich insbesondere zur Herstellung einer Endoprothese, zum Beispiel in Form eines selbstexpandierenden oder ballonexpandierbaren Stents, eignet. Der Einsatz von biokorrodierbaren metallischen Werkstoffen in Implantaten dürfte zu einer deutlichen Minderung von Abstoßungs- oder Entzündungsreaktionen führen. Solche biokorrodierbaren Implantate und Stents weisen häufig auch eine Beschichtung oder Kavitätenfüllung mit einem geeigneten Polymer auf.

Allerdings weist eine Vielzahl dieser biokorrodierbarer Implantatwerkstoffe aufgrund der hohen mechanischen Belastung und/oder der unter anderem von den Abbauprodukten des Werkstoffs hervorgerufenen Vasospasmen eine verringerte mechanische Integrität auf, was dazu führt, dass die Implantate früher als erforderlich ihre Stützfähigkeit verlieren.

Eine weitere Möglichkeit, die Risikofaktoren einer Restenose zu verhindern, stellt die Entwicklung einer Vielzahl an Beschichtungen für Stents dar, die eine erhöhte Hämokompatibilität bieten sollen. Seit längerer Zeit werden bspw. in die Beschichtung der Stents vasodilatierende, antikoagulierende, antimikrobielle, antiinflammatorische und antiproliferative Agenzien sowie Wirkstoffe, welche das Restenoserisiko vermindern oder gar verhindern, einzeln oder in Kombination eingesetzt. Diese Substanzen sollen aus dem Beschichtungsmaterial des Stents derart freigesetzt werden, dass sie Entzündungen des umliegenden Gewebes, überschießendes Wachstum der glatten Muskelzellen, das Verklumpen von Blut oder Vasospasmen des umliegenden Implantationsgewebes verhindern.

Derartige beschichtete Stents sind dem Fachmann bekannt. Beispielsweise werden in den Patentanmeldungen US 2004/0148013 und US 2007/0048351 und im Patent US 7,323,189 beschichtete medizinische Vorrichtungen wie Stents offenbart, wobei aus der Beschichtung Wirkstoffe oder Wirkstoffkombinationen freigesetzt werden können. Unter anderem kommen hier Statine zum Einsatz.

Allerdings haben viele dieser beschichteten Stents den Nachteil, dass für eine erfolgreiche Therapie am Implantationsgewebe die jeweiligen Wirkstoffe aufgrund ihrer eingeschränkten biologischen Eigenschaften entweder in einer erhöhten Konzentration eingesetzt werden müssen, was zu einer lokalen Intoxikation führen kann, oder in Kombination mit anderen Wirkstoffen auf die Beschichtung auf- oder eingebracht werden müssen, was erhöhte Produktionsaufwendungen und -kosten zur Folge hat.

Es ist daher wünschenswert, Wirkstoffe zu entwickeln, die eine verbesserte pharmakologische Wirksamkeit aufweisen und die in der Lage sind, bestimmte physikalische, chemische oder biologische Prozesse, die die unerwünschte Körperreaktion hervorrufen, zu inhibieren und diese somit für einen gewünschten Zeitraum zu vermindern oder besten Falls vollständig zu vermeiden.

Das Patent US 7,166,638 offenbart neue Derivate des Statins, Nitro-Statine oder Statin-Nitroderivate, welche ein deutlich verbessertes Gesamtprofil im Vergleich zu natürlichen Statinen hinsichtlich einer breiteren pharmakologischen Wirksamkeit. Insbesondere wurde erkannt, dass bei einer systemischen Verabreichung einer Lösung aus Nitro-Statinen, diese Verbindungen eine starke entzündungshemmende, anti-thrombotische und Antiplättchen-Wirksamkeit besitzen und außerdem zur Reduzierung der Cholesterin- und Triglyceridspiegel, zur Anhebung der HDL-C-Spiegel und zur Behandlung und Vorbeugung von akuten Koronarsyndromen, Schlaganfall, peripherarteriosklerotischen Gefäßerkrankungen und allen mit endothelialen Disfunktionen verbundenen Störungen wie z.B. vaskulären Beschwerden bei zuckerkranken Patienten und Arteriosklerose verwendet werden können. Sie weisen weiterhin geeignete Eigenschaften zur Behandlung neurodegenerativer und Autoimmunkrankheiten wie z.B. Alzheimer-Krankheit und Parkinson-Krankheit sowie Multipler Sklerose auf.

Allerdings besitzt eine systemische Verabreichung von Wirkstoffen den Nachteil, dass die Wirkstoffe nicht gezielt an das zu therapierende Gewebe abgegeben werden und somit in der Regel eine längerfristige und mehrmalige Wirkstoff Verabreichung vor, während und/oder nach der Implantation notwendig ist.

Aufgabe der vorliegenden Erfindung war es, einen oder mehrere der geschilderten Nachteile des Standes der Technik zu mindern oder zu überwinden.

Diese Aufgabe wird erfindungsgemäß dadurch gelöst, dass ein medizinisches Implantat ganz oder in Teilen mit einer Beschichtung, die aus mindestens einem Wirkstoff besteht oder mindestens einen Wirkstoff enthält, überzogen ist, wobei der Wirkstoff ein Nitro-Statin ist.

Ein Vorteil der erfindungsgemäßen Lösung liegt darin, dass Nitro-Statine gezielt am Implantationsort antithrombotisch, antiinflammatorisch und antiproliferativ wirken.

Überraschend war, dass Nitro-Statine bedingt durch die NO-Freisetzung in das umliegende Implantationsgewebe zusätzlich eine ausgezeichnete vasodilatierende Wirkung besitzen, so dass einer Vasokonstriktion direkt vor Ort durch die Freisetzung des Wirkstoffs aus der Beschichtung des erfindungsgemäßen Implantats entgegengewirkt wird. Diese Eigenschaft der erfindungsgemäßen Implantate ist dann besonders vorteilhaft, wenn es sich um ein Implantat aus einem biokorrodierbaren Werkstoff handelt, weil somit längere Standzeiten der Implantate erreicht werden können und, wenn das erfindungsgemäße Implantat z.B. ein Stent ist, die Gefahr einer Restenose bzw. der Beeinträchtigung des Gefäßlumens im und um den Stentbereich deutlich verringert werden kann. Somit kann der mit dem Einsatz des Implantats verfolgte Therapiezweck besser, über einen längeren Zeitraum und mit weniger Nebenwirkungen erreicht werden.

Medizinische Implantate innerhalb des Schutzumfangs der vorliegenden Erfindungen beinhalten beliebige medizinische Vorrichtungen, welche benutzt werden, wenigstens teilweise, um in den Körper eines Patienten eingebracht zu werden. Beispiele beinhalten implantierbare Vorrichtungen Herzschrittmacher, Katheter, Nadelinjektionskatheter, Blutgerinnselfilter, vaskuläre Transplantate, Ballons, Stenttransplantate, Gallenstents, Darmstents, Bronchiallungenstents, Speiseröhrenstents, Hamleiterstents, Aneurysmenausfüllende Spulen und andere Spulenvorrichtungen, transmyokardiale Revaskularisationsvorrichtungen, perkutane myokardiale Revaskularisationsvorrichtungen. Weiterhin können beliebige natürliche und/oder künstliche Medizinprodukte eingesetzt werden, beispielsweise Prothesen, Organe, Gefäße, Aorten, Herzklappen, Schläuche, Organersatzteile, Implantate, Fasern, Hohlfasern, Membranen, Konserven, Blutbehältern, Titerplatten, Adsorbermedien, Dialysatoren, Verbindungsstücke, Sensoren, Ventile, Endoskope, Filter, Pumpenkammern sowie andere Medizinprodukte, welche hämokompatible Eigenschaften aufweisen sollen. Der Begriff Medizinprodukte ist weit zu fassen und bezeichnet insbesondere solche Produkte, die kurzzeitig (z.B. Endoskope) oder dauerhaft (z.B. Stents) mit Blut in Kontakt kommen, auch Kombination einzelner medizinsicher Implantate, beispielsweise Stent und Ballonkatheter, aber ebenso implantierbare Defibrillatoren, Herzschrittmacher, Schrittmacherelektroden wie auch diagnostische Katheter.

Besonders bevorzugte Medizinprodukte sind Ballonkatheter und Stents.

Stents herkömmlicher Bauart weisen eine filigrane Stützstruktur aus metallischen Streben auf, die zur Einbringung in den Körper zunächst in einem nicht-expandierten Zustand vorliegen und die am Ort der Applikation dann in einen expandierten Zustand aufgeweitet werden. Der Stent kann vor oder nach dem Crimpen auf einen Ballon beschichtet werden.

Der Grundkörper des Stents besteht vorzugsweise aus einem metallischen Material aus einem oder mehreren Metallen aus der Gruppe Eisen, Magnesium, Nickel, Wolfram, Titan, Zirkonium, Niob, Tantal, Zink oder Silizium und ggf. einer zweiten Komponente aus einem oder mehreren Metallen aus der Gruppe Lithium, Natrium, Kalium, Kalzium, Mangan, Eisen oder Wolfram, vorzugsweise aus einer Zink-Kalziumlegierung.

In einem weiteren Ausführungsbeispiel besteht der Grundkörper aus einem Formgedächtnis-Material aus einem oder mehreren Materialien aus der Gruppe bestehend aus Nickel-Titan-Legierungen und Kupfer-Zink-Aluminium-Legierungen, vorzugsweise aber aus Nitinol.

In einem weiteren bevorzugten Ausführungsbeispiel besteht der Grundkörper des Stents aus Edelstahl, vorzugsweise aus einem Cr-Ni-Fe-Stahl - hier bevorzugt die Legierung 316L - oder einem Co-Cr-Stahl. Ferner kann der Grundkörper des Stents mindestens teilweise aus Kunststoff und/oder einer Keramik bestehen.

In einem weiteren Ausführungsbeispiel besteht der Grundkörper des Stents aus einem biokorrodierbaren metallischen Werkstoff, zum Beispiel einer biokorrodierbaren Legierung, ausgewählt aus der Gruppe Magnesium, Eisen und Wolfram; insbesondere ist der biokorrodierbare metallische Werkstoff eine Magnesiumlegierung.

Unter einer biokorrodierbaren Magnesiumlegierung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

In einem weiteren Ausführungsbeispiel besteht der Stent aus natürlichen Polymeren, wie zum Beispiel aus Collagen, Chitin, Chitosan, Heparin.

Ballonkatheter in Rahmen der vorliegenden Erfindung werden in vielen Bereichen der Medizintechnik eingesetzt. Der Einsatz von Ballonkathetem ist ein bevorzugtes therapeutisches Verfahren bei verschiedenen Indikationen. So wird beispielsweise in der Angiologie und Kardiologie eine Aufweitung verengter Blutgefäße mittels Ballondilatation vorgeschlagen.

Die Grundkonstruktion von Ballonkathetem ist beispielsweise aus den Druckschriften US 5,522,882 A und US 7,217,278 B2 bekannt ist. So weisen Ballonkatheter, wie sie etwa für das Aufweiten von krankhaft verengten Gefäßen im Körper oder für das Setzen von Gefäßwandstützen - sogenannter "Stents" - eingesetzt werden, einen Außenschaft mit einem distalen Ende und einen darin unter Bildung einer ringförmigen Fluidleitung angeordneten Innenschaft auf, der über das distale Ende des Außenschaftes hinaussteht. Am distalen Ende des Katheters ist ein Ballon mit einer ersten Befestigungszone an seinem proximalen Ende fluiddicht auf dem distalen Endbereich des Außenschaftes und mit einer zweiten Befestigungszone an seinem distalen Ende fluiddicht auf dem distalen Endbereich des Innenschaftes befestigt. Zwischen diesen Befestigungszonen ist der Ballon in undilatiertem Zustand in Längsfalten gelegt, damit sein Außendurchmesser in diesem Zustand möglichst gering ist. Dies ist Voraussetzung dafür, dass der Ballonkatheter mit dem Ballon am distalen Ende auch durch enge Gefäße oder stark gekrümmte Gefäßbereiche vorgeschoben werden kann. Nach der Platzierung des Ballons an seinem Einsatzort kann dann über die zwischen Innen- und Außenschaft gebildete ringförmige Fluidleitung ein Fluid unter Druck eingebracht und der Ballon dilatiert werden. Dabei entfalten sich die Längsfalten in Peripherrichtung unter starker Durchmesservergrößerung des Ballons.

Typische Ballonmaterialien sind meist semikristalline Thermoplaste, wobei im PTCA-/PTA-Bereich primär Polyamide, Polyethylenterephthalat (PET), Polybutylenterephthalat (PBT) oder Polyether-Polyurethane sowie deren Copolymere und Blends eingesetzt werden. Polyurethane setzen sich als alternative Materialien zunehmend für dehnbare und anpassungsfähige Ballonapplikationen durch.

Eine Beschichtung im Sinne der Erfindung ist eine zumindest abschnittsweise Auftragung der Komponenten der Beschichtung auf den Grundkörper des Implantats. Vorzugsweise wird die gesamte Oberfläche des Grundkörpers des Implantats von der Beschichtung bedeckt. Die Schichtdicke liegt vorzugsweise im Bereich von 1 nm bis 100 µm, besonders bevorzugt 300 nm bis 15 µm und ganz besonders bevorzugt von 5 µm bis 60 µm. Die Beschichtung kann ein Polymer enthalten, welches eine Trägermatrix bilden kann. Der Gewichtsanteil einer solchen Polymermatrix an den die Beschichtung bildenden Komponenten der Beschichtung beträgt vorzugsweise mindestens 40%, besonders bevorzugt mindestens 70%. Der Gewichtsanteil des mindestens einen Nitro-Statins an den die Beschichtung bildenden Komponenten der Beschichtung beträgt bevorzugt nicht mehr als 60%, besonders bevorzugt nicht mehr als 30% und ganz besonders bevorzugt liegt er im Bereich von 10% bis 25%. Die Beschichtung kann direkt auf die Implantatoberfläche aufgetragen werden. Die Verarbeitung kann nach Standardverfahren für die Beschichtung erfolgen. Es können einschichtige, aber auch mehrschichtige Systeme (zum Beispiel sogenannte base coat-, drug coat- oder top coat-Schichten) erstellt werden. Die Beschichtung kann unmittelbar auf dem Grundkörper des Implantats aufgebracht sein oder es sind weitere Schichten dazwischen vorgesehen, die beispielsweise der Haftvermittlung dienen.

Alternativ kann die Beschichtung, bestehend aus oder enthaltend mindestens ein Nitro-Statin, als Kavitätenfüllung vorliegen oder Bestandteil einer Kavitätenfüllung sein. Das Implantat, insbesondere der Stent, weist zu diesem Zweck eine oder mehrere Kavitäten auf. Kavitäten befinden sich beispielsweise an der Oberfläche des Implantats und lassen sich beispielsweise durch Laserablation in Nano- bis Mikrometerdimensionen erstellen. Bei Implantaten, insbesondere Stents, mit einem biodegradierbaren Grundkörper kann eine Kavität auch im Innern des Grundkörpers angeordnet sein, so dass die Freisetzung des Material erst nach Freilegung erfolgt. Der Fachmann kann sich bei der Ausgestaltung der Kavität an dem im Stand der Technik beschriebenen Systemen orientieren. Der Begriff "Kavität" umfasst dabei z.B. Löcher und Vertiefungen.

Die Ausführungsform einer Beschichtung als Kavitätenfüllung ist dann besonders interessant, wenn größere Mengen des mindestens einen Nitro-Statins über einen längeren Zeitraum an das Implantationsgewebe abgegeben werden soll. Durch die Befüllung von Kavitäten kann die Beladung an Wirkstoff auf dem Implantat um 50% erhöht werden.

Bevorzugt liegt das mindestens eine Nitro-Statin in einer polymeren Trägermatrix eingebettet vor. Die Trägermatrix im Rahmen der vorliegenden Erfindung ist eine biostabile und/oder bioabbaubare Polymerschicht, wobei die Polymere ausgewählt sind aus der Gruppe bestehend aus nichtresorbierbaren, permanenten Polymeren und/oder resorbierbaren bioabbaubaren Polymeren.

Besonders bevorzugt ist die Trägermatrix zusammengesetzt aus Polymeren ausgewählt aus der Gruppe Polyolefine, Polyetherketone, Polyether, Polyvinylalkohole, Polyvinylhalogenide, Polyvinylester, Polyvinylpyrrolidon, Polyacrylate, Polyhalogenolefine, Polyamide, Polyamidimide, Polysulfone, Polyester, Polyvinylether, Polyurethane, Silikone, Polyphosphazene, Polyphenylen, Polymerschäume (aus Styrolen und Carbonaten), Polydioxanone, Polyglycolide, Polylactide, Poly-ε-caprolacton, Ethylvinylacetat, Polyethylenoxid, Polyphosphorylcholin, Polyhydroxybuttersäuren, Lipide, Polysaccharide, Proteine, Polypeptide sowie Copolymere, Blends und Derivate dieser Verbindungen.

Ganz besonders bevorzugt ist die Trägermatrix zusammengesetzt aus Polymeren ausgewählt aus der Gruppe bestehend aus Polypropylen, Polyethylen, Polyisobutylen, Polybutylen, Polyetheretherketon, Polyethylenglycol, Polypropylenglycol, Polyvinylalkohole, Polyvinylchlorid, Polyvinylfluorid, Polyvinylacetat, Poly(vinylisobutylether), Polyvinylpyrrolidon, Polyethylacrylat, Polymethylacrylat, Poly(methylmethacrylat), Poly(ethylmethacrylat), Poly(butylmethacrylat), Poly(isobutylmethacrylat), Poly(hexylmethacrylat), Poly(phenylmethacrylat), Poly(isopropylacrylat), Poly(isobutylacrylat), Poly(octadecyacrylat), Polytetrafluorethylen, Polychlortrifluorethylen, PA 11, PA 12, PA 46, PA 66, Polyamidimide, Polyethersulfon, Polyphenylsulfon, Polycarbonate, Polybutylenterephthalat, Polyethylenterephthalat, Elastane, Pellethane, Silikone, Polyphosphazene, Polyphenylen, Polymerschäume (aus Styrolen und Carbonaten), Polydioxanone, Polyglycolide, Poly-L-, Poly-D-, und Poly-D,L-Lactid, sowie Poly-ε-caprolacton, Ethylvinylacetat, Polyethylenoxid, Polyphosphorylcholin, Polyhydroxyvalerat, Cholesterin, Cholesterinester, Alginat, Chitosan, Levan, Hyaluronsäure, Uronide, Heparin, Dextran, Methylcellulose, Ethylcellulose, Hydroxypropylcellulose, Hydroxypropylmethylcellulose, Hydroxybutylmethylcellulose, Celluloseacetat, Cellulosepropionat (mit niedrigem, mittlerem oder hohem Molekulargewicht), Celluloseacetatpropionat, Celluloseacetatbutyrat, Celluloseacetatphthalat, Carboxymethylcellulose, Cellulosetriacetat, Cellulosesulfatnatriumsalz, Fibrin, Albumin, Polypeptide und Copolymere, Blends und Derivate dieser Verbindungen.

Die Trägermatrix richtet sich bevorzugt nach der gewünschten Elutionsgeschwindigkeit sowie den individuellen Charakteristika der verschiedenen eingesetzten Wirkstoffe und nach der unterschiedlichen Resorptions- bzw. Degradationsgeschwindigkeit am Wirkort des medizinischen Implantats.

In einer bevorzugten Ausführungsform erfolgt die Wirkstoffabgabe aus der Beschichtung der Implantatoberfläche, die mindestens ein Nitro-Statin enthält, in das umliegende Implantationsgewebe zwischen 2 Sekunden und mehr als 2 Wochen, besonders bevorzugt zwischen 2 Sekunden und mehr als 6 Wochen.

In einer weiteren bevorzugten Ausführungsform erfolgt die Wirkstoffabgabe aus der Beschichtung von einem Ballonkatheter in das umliegende Implantationsgewebe zwischen 1 und 90 Sekunden, besonders bevorzugt zwischen 1 und 30 Sekunden. Hier kann zusätzlich in die Beschichtung ein pharmazeutischer Hilfsstoff auf- und/oder eingebracht werden.

Bei einem pharmazeutischen Hilfsstoffen im Rahmen der vorliegenden Erfindung handelt es sich beispielsweise um Benzalkoniumchlorid, α-Tocopherol, Glucose, Lactose, Calciumphosphat, Calciumhydrogenphosphat, Natriumhydrogencarbonat, Natriumcarbonat, Titanoxid, Zinkoxid, Magnesiumoxid, Silikate wie hochdisperses Siliciumdioxid (kolloidale Kieselsäure, Aerosil, SiO₂), Talkum, Kaolin, Bentonit, aliphatische Alkohole, DMSO, Glycerol, Propylenglykol, Stearinsäure, Zucker und Zuckeralkohole, Lactose, Cyclodextrine wie α-Cyclodextrin, β-Cyclodextrin, γ-Cyclodextrin, Mannitol, Sorbitol, Stärken, Cellulosepulver, Celluloseester und -ether wie Methylcellulose, Ethylcellulose, Hydroxyethylcel-lulose, Hydroxypropylcellulose, Hydroxypropylmethylcellulose, Natriumcarboxymethylcellulose, Celluloseacetatphthalat, Hydroxypropylmethyl-celluloseacetatphthalat und mikrokristalline Cellulose, Gelatine, Squalen und andere Isopren-Einheiten, Gummi arabicum, Pektin, Xanthan, Alginate, Schellack, Polyacrylsäuren wie Carbomere und Eudragit®, Polyvinylpyridin, Polyvinylpyrrolidon, Polyvinylalkohol, Polyvinylacetat und Mischungen aus Polyvinylpyridin und Polyvinylacetat, Polyethylenglykol, Vaselin, synthetische und natürliche Fette und Silikone, amphiphile oder oberflächenaktive Hilfsstoffe, wie anionenaktive Tenside, Saponide; kationische Tenside wie Cetylpyridiniumchlorid, nichtionogene Tenside; Polyoxyethylensorbitan, Macrogolglycerolfettsäureester, Fettalkoholether des Polyoxyethylens, Fettsäureester der Saccha-rose und insbesondere D-α-Tocopheryl-1000-succinat, amphotere Tenside, Komplexemulgatoren wie Cetylstearylalkohol (Typ A und B), quartäre Ammoniumverbindungen und Konservierungsmittel und Antioxidantien wie Citronensäure, Citraconsäure, Weinsäure, Mono- und Poly-phosphate, organische Phosphate, wie Dodecylphosphat, Hexosephosphat und Hyaluronidasen oder Hyaluronatlyasen, Butyryl-n-trihexylcitrat (BTHC) und Triethylcitrat. Der Gewichtsanteil eines solchen pharmazeutischen Hilfsstoffs an den die Beschichtung bildenden Komponenten der Beschichtung beträgt vorzugsweise mindestens 0,1 %, besonders bevorzugt mindestens 2 %.

Das mindestens eine Nitro-Statin im Rahmen der vorliegenden Erfindung umfasst eine Verbindung der allgemeinen Formel (I) und ein pharmazeutisch annehmbares Salz oder Stereoisomer davon 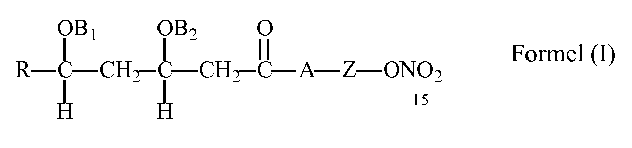 worin R der unten dargestellte Statin-Rest (auch Statin-Komponente) ist, A wie unten definiert ist und Z eine der unten definierten geeignete Verknüpfungsgruppe ist.

Erfindungsgemäß haben R, B₁, B₂, Z und A in der allgemeinen Formel (I) die folgenden Bedeutungen:
R ist 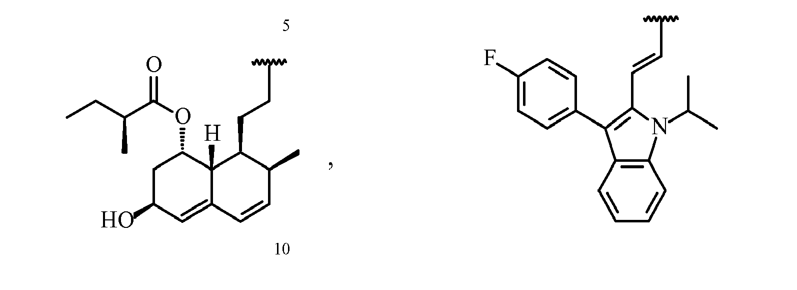 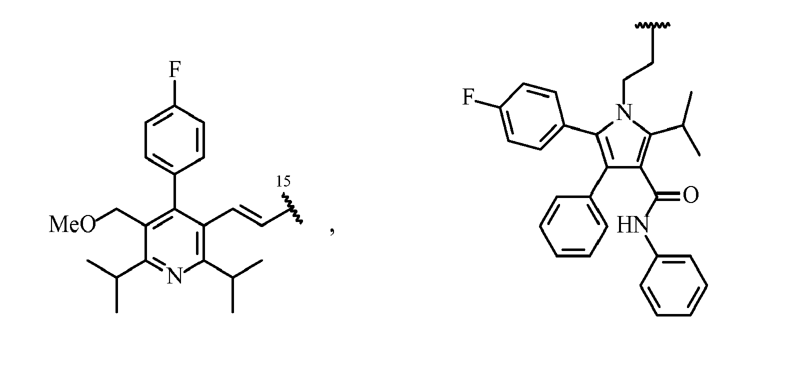 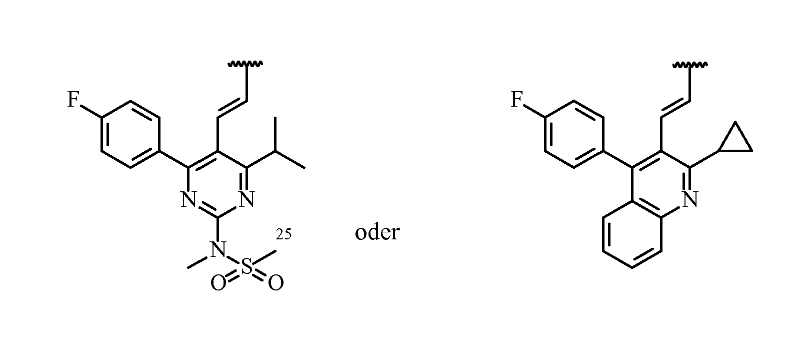
B₁ und B₂ ist a) eine weitere NO-freisetzende Gruppe wie -NO₂, -ONO₂ oder Cinidomin (II) 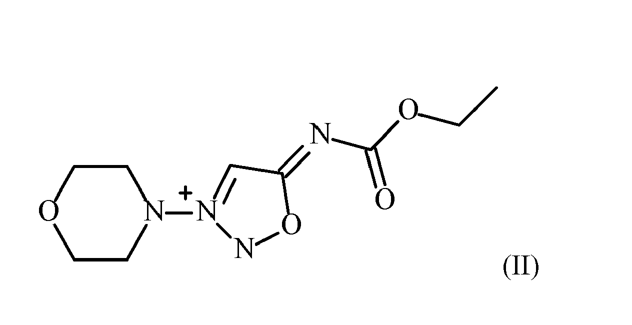
   b) eine Tosylat- oder Mesylat-Gruppe
   c) ein geradkettiges oder verzweigtes C₁ bis C₂₀-Alkyl, bevorzugt C₁ bis C₁₀-Alkyl, das optional mit einem oder mehreren Substituenten substituiert ist, die aus der Gruppe ausgewählt werden, die aus folgenden bestehen: Halogenatomen, Hydroxy-,-ONO,
   d) ein heterocyclischer gesättigter, ungesättigter oder aromatischer Ring, der ein oder mehrere Heteroatome enthält, die aus Stickstoff, Sauerstoff, Schwefel oder Halogenatomen ausgewählt werden, wobei der Ring zusätzlich mit Seitenketten substituiert ist, die ein geradkettiges oder verzweigtes Alkyl mit 1 bis 10 Kohlenstoffatomen aufweisen.
   e) Cycloalkylen oder Cycloarylen mit 5 bis 7 Kohlenstoffatomen im Cycloalkylen- oder Cycloarylenring, wobei der Ring mit Seitenketten substituiert ist, die ein geradkettiges oder verzweigtes Alkyl mit 1 bis 10 Kohlenstoffatomen aufweisen, und optional ein oder mehrere Heteroatome enthält, die aus Stickstoff, Sauerstoff, Schwefel oder Halogenatomen ausgewählt werden,
   wobei B₁ gleich oder verschieden B₂.

A ist ein geradkettiges oder verzweigtes C₁ bis C₂₀-Alkylen, bevorzugt C₁ bis C₁₀-Alkyl, insbesondere -CH₂-; -O-, -S-, -NH- oder NR¹, worin R¹ ein geradkettiges oder verzeigtes C₁-C₁₀-Alkyl, bevorzugt CH₃, ist; ganz besonders bevorzugt ist A: -CH₂-, -O- oder -S-.; Z ist ein zweiwertiger Rest mit folgender Bedeutung:
a) geradkettiges oder verzweigtes C₁-C₂₀-Alkylen, bevorzugt C₁-C₁₀-Alkylen, das optional mit einem oder mehreren Substituenten substituiert ist, die aus der Gruppe ausgewählt werden, die aus folgenden bestehen: Halogenid, -OH, -OR¹, -COOH, -COOR¹, -NH₂, NHR¹, NR¹R², worin R¹ und R² gleich oder verschieden sind und ein geradkettiges oder verzeigtes C₁-C₁₀-Alkyl sind; b) Cycloalkylen mit 5 bis 7 Kohlenstoffatomen im Cycloalkylenring, worin der Cycloalkylenring optional mit einer oder mehreren geradkettigen oder verzeigten C₁-C₁₀-Alkyl-Seitenketten substituiert ist.
c) Arylalkylen, insbesondere 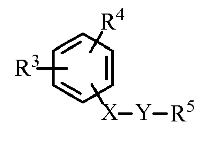 worin R³ gleich (CH₂)ₙ₁, worin n1 = 0 oder 20; R⁴ eine -COOH, -COOR¹, -OH, -OR¹ ist, worin R¹ wie oben definiert ist; X eine gesättigte oder ungesättigte -(CH₂)ₘ-Gruppe ist, worin m = 0-8; Y = -O-CO-, -COO-; R⁵ gleich (CH₂)ₙ₂, worin n2 = 1-20.
d) Heterocycloalkylen mit einem gesättigten, ungesättigten oder aromatischen 5- oder 6-gliedrigen Ring, der ein oder mehrere Heteroatome enthält, die aus Stickstoff, Sauerstoff, 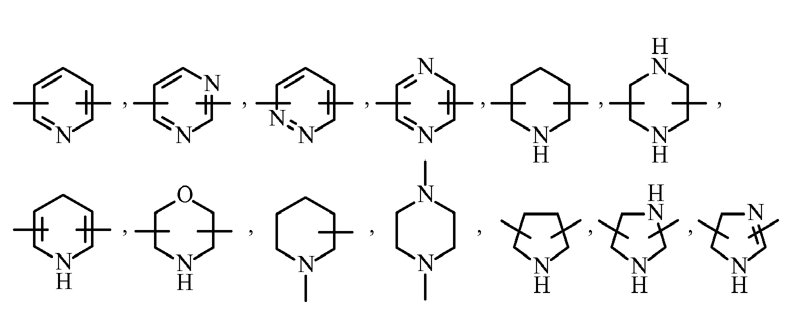 Schwefel ausgewählt werden, insbesondere folgende Gruppen:
e) -O-, -Sᵢ-, -Se-, -NH- oder NR¹, worin i eine Zahl zwischen 1 bis 8 ist und R¹ ein geradkettiges oder verzeigtes C₁-C₁₀-Alkyl, bevorzugt CH₃, ist.

Insbesondere wird der Statin-Rest R in der allgemeinen Formel (I) aus der Gruppe ausgewählt, die aus Atorvastatin, Rosuvastatin, Pravastatin, Fluvastatin, Lovastatin, Pitavastatin, Simvastatin und Cerivastatin besteht.

Folgende Gruppen R, A und Z sind besonders:
A CH₂, O, S
Z O, S

Verandwortlich für die NO-Freisetzung sind bei vielen Nitrostatinen die Nitratester der folgenden Formel:
R-O-N⁺(O)-O⁻. dieses häufig auftretende Strukturmotiv ist besonders für eine NO Abspaltung geeignet.

Das Cinidomin als NO freisetzende Verbindung nimmt eine Sonderstellung ein. Hier liegt ein Prodrug vor, welches in der Leber umgewandelt wird. Dieser Metabolit spaltet dann NO ohne Enzymkatalyse ab.

Die Verbindungsklasse der Nitro-Statine weist auf den erfindungsgemäßen medizinischen Implantaten herausragende Eigenschaften auf. Dies liegt in der speziellen Struktur dieser Verbindungen, die in 2 hochpotente Komponenten aufgeteilt werden können: ein Statin-Rest und eine bei der Metabolisierung gebildete NO-Komponente. Somit besitzen Nitro-Statine ein breites pharmakologisches Wirkspektrum.

Die Herstellung von Nitro-Statinen ist dem Fachmann bekannt und soll hier nur kurz behandelt werden. Statine können, wenn sie eine Carbonylfunktion aufweisen, in einfacher Form zu Nitro-Statinen umgesetzt werden. Halogenide können sehr elegant mit sehr guten Ausbeuten mit NO₂⁺BF₄⁻ zum Nitro-Statin umgesetzt werden. Alternativ mit weniger milden Bedingungen ist die Umsetzung mit Silbernitrat durchführbar. Um Halogenide zu erhalten eignet sich eine HunsdieckerReaktion, bei der die -COOH Gruppe mit Ag⁺ in CCl₄ umgesetzt wird, anschließendes Quenschen mit Br₂ ergibt das Halogenid. Alkohole können noch einfacher in die Nitratestergruppe überführt werden. Hier eignet sich die Umsetzung mit HNO₃, evtl. mit HNO₃ und H₂SO₄, der Nitriersäure. Dieser Reaktionsweg ist wegen der auftretenden Nebenprodukte weniger geeignet, als die Überführung in die Alkylhalogenide und die folgende Umsetzung mit Silbernitrat AgNO₃ oder dem bevorzugtem NO₂⁺BF₄⁻. Neben der beschriebenen Hunsdiecker-Reaktion zur Gewinnung von Halogeniden ist auch die Variante der Umsetzung der Säuregruppe mit HgO in Gegenwart von Br₂ machbar. Chlorhalogenide lassen sich über die Kochi-Reaktion gewinnen. Die -COOH Gruppe wird mit Litiumchlorid (LiC1) in Gegenwart von Bleitetraacetat (Pb(OAc)₄) umgesetzt.

Der Zugang zu diesen Derivaten ist in Lehrbüchern der organischen Chemie beschrieben.

Die Statin-Komponente besitzt antithrombotische, antiinflammatorische und antiproliferative Eigenschaften, die bei der Behandlung von gereiztem oder leicht verletztem Implantationsgewebes von besonderer Bedeutung sind. Zusätzlich ist bekannt, dass Statine eine Cholesterolsynthese-Hemmung bewirken und damit eine Verlangsamung der lokalen Progredienz der Arteriosklerose bewirken. Weiterhin fördern Statine die positive Zusammensetzung der Plaque bzw. Plaquevorstufen sowie eine verstärkte Rekrutierung endothelialer Vorläuferzellen aus dem Knochenmark, was dazu führt, dass die Wiederherstellung des Endothels am Implantationsgewebe beschleunigt wird, wodurch wiederum die verletzungsbedingte Neointimabildung unterdrückt wird.

Die freigesetzte NO-Komponente in das umliegende Implantationsgewebe besitzt eine ausgezeichnete vasodilatierende Wirkung, so dass einer Vasokonstriktion direkt vor Ort durch die Freisetzung des Wirkstoffs aus der Beschichtung des erfindungsgemäßen Implantats entgegengewirkt wird. Diese Eigenschaft ist insbesondere vorteilhaft, wenn das medizinische Implantat aus einem biokorrodierbaren Werkstoff, wie Magnesium oder eine Legierung hiervon, besteht. Beim Abbau der biokorrodierbaren Magnesiumlegierung bewirken deren Abbauprodukte eine Alkalose am Implantationsort, wodurch lokale Vasospasmen auftreten können. Zusätzlich zu der mechanischen Belastung bewirkt eine Vasokonstriktion eine oft nicht erwünschte verkürzte Standzeit des Implantats. Durch die vasodilatierende Wirkung der NO-Komponente können längere Standzeiten der Implantate erreicht werden. Weiterhin vorteilhaft ist die Verwendung eines Nitro-Statins, das mehrere NO-freisetzende Gruppen aufweist. Somit kann die vasodilatierende Wirkung verstärkt und auf den entsprechenden Implantationsort, der das Implantat möglicherweise stärker beanspruchten, angepasst werden.

Falls es sich beim erfindungsgemäßen Implantat um einen Stent handelt, kann die Gefahr einer Restenose bzw. der Beeinträchtigung des Gefäßlumens im und um den Stentbereich deutlich verringert werden. Somit kann der mit dem Einsatz des Implantats verfolgte Therapiezweck besser, über einen längeren Zeitraum und mit weniger Nebenwirkungen erreicht werden.

Das mindestens eine Nitro-Statin ist bevorzugt in einer pharmazeutisch aktiven Konzentration von 0,2 - 3,5 µg/mm² Implantatoberfläche, weiter bevorzugt von 0,5 - 1,6 µg/mm² Implantatoberfläche enthalten.

Zusätzlich zu dem mindestens einen Nitro-Statin kann das erfindungsgemäße medizinische Implantat einen oder mehrere weitere pharmazeutische Wirkstoffe umfassen, die an die Umgebung des Implantats abgegeben werden und gegebenenfalls mit sehr niedrigen Raten in den Blutkreislauf freigesetzt werden. Der weitere pharmazeutische Wirkstoff ist bevorzugt aus den folgenden Arzneistoffklassen ausgewählt: antimikrobielle, antimitotische, antimyotische, antineoplastische, antiphlogistische, antiproliferative, antithrombotische und vasodilatatorische Mittel.

Besonders bevorzugte weitere pharmazeutische Wirkstoffe sind Triclosan, Cephalosporin, Aminoglycosid, Nitrofurantoin, Penicilline wie Dicloxacillin, Oxacillin sowie Sulfonamide, Metronidazol, 5-Fluoruracil, Cisplatin, Vinblastin, Vincristin, Epothilone, Endostatin, Verapamil, Statine wie Cerivastatin, Atorvastatin, Simvastatin, Fluvastatin, Rosuvastatin, Pravastatin sowie Lovastatin, Angiostatin, Angiopeptin, Taxane wie Paclitaxel, Immunsuppressiva oder -modulatoren wie z.B. Rapamycin oder dessen Derivate wie Biolimus, Everolimus, Ridaforolimus (vormals als Deforloimus bekannt), Novolimus, Myolimus, Temsirolimus, Methotrexat, Colchicin, Flavopiridol, Suramin, Cyclosporin A, Clotrimazol, Flucytosin, Griseofulvin, Ketoconazol, Miconazol, Nystatin, Terbinafin, Steroiden wie Dexamethason, Prednisolon, Corticosteron, Budesonid, Östrogen, Hydrocortison sowie Mesalamin, Sulfasalazin, Heparin und seinen Derivaten, Urokinase, PPack, Argatroban, Aspirin, Abciximab, synthetisches Antithrombin, Bivalirudin, Enoxoparin, Hirudin, r-Hirudin, Protamin, Prourokinase, Streptokinase, Warfarin, Flavonoiden wie 7,3',4'-trimethoxyflavon sowie Dipyramidol, Trapidil sowie Nitroprusside.

Der weitere pharmazeutische Wirkstoff wird einzeln oder kombiniert in gleicher oder unterschiedlicher Konzentration eingesetzt.

Der weitere pharmazeutische Wirkstoff ist bevorzugt in einer pharmazeutisch aktiven Konzentration von 0,2 bis 3,5 µg/mm² Implantatoberfläche, weiter bevorzugt von 0,5 bis 2,4 µg/mm² Implantatoberfläche enthalten.

Besonders bevorzugt ist eine Kombination des mindestens einen Nitro-Statins mit einem oder mehreren antiproliferativen Wirkstoffen. Insbesondere bevorzugt ist die Kombination des mindestens einen Nitro-Statins mit mindestens einem der weiteren pharmazeutischen Wirkstoffe Paclitaxel und Rapamycin, einzeln oder in Kombination.

Weiterhin bevorzugt ist eine Kombination des mindestens einen Nitro-Statins mit einem oder mehreren Wirkstoffen, die den mTOR-Weg beeinflussen. Insbesondere bevorzugt ist die Kombination des mindestens einen Nitro-Statins mit mindestens einem der weiteren pharmazeutischen Wirkstoffe Sirolimus, Everolimus, Biolimus A9, Myolimus, Novolimus, Zotarolimus oder andere verwandte Wirkstoffe, sowie nukleinsäurebasierte Wirkstoffe die beispielsweise via RNA-Interferenz den mTOR-Weg beeinflussen einzeln oder in Kombination. Die Beeinflussung des mTOR-Weges ermöglicht eine effiziente Modifikation der proliferativen Reaktion der Gefäßwand auf das Implantat. Der zusätzliche besondere Vorteil einer Kombination mindestens eines mTOR-beeinflussenden Wirkstoffes mit mindestens einem Nitro-Statin liegt in der synergistischen antiproliferativen und antiinflammatorischen Wirkung, supplementiert durch antithrombotische und vasodilatierende Wirkungen.

Weiterhin bevorzugt ist eine Kombination des mindestens einen Nitro-Statins mit einem oder mehreren Wirkstoffen, die mit Mikrotubuli oder deren Bestandteilen interagieren. Insbesondere bevorzugt ist die Kombination des mindestens einen Nitro-Statins mit mindestens einem der weiteren pharmazeutischen Wirkstoffe Paclitaxel, Docetaxel und andere Taxane und verwandte Wirkstoffe, Wirkstoffe aus der Gruppe der Epothilone, sowie nukleinsäurebasierte Wirkstoffe die beispielsweise via RNA-Interferenz Synthese, Aufbau, Struktur oder Abbau der Mikrotubuli beeinflussen einzeln oder in Kombination. Die Beeinflussung der Mikrotubuli ermöglicht eine effiziente Modifikation der proliferativen Reaktion der Gefäßwand auf das Implantat. Der zusätzliche besondere Vorteil einer Kombination mindestens eines Mikrotubuli-beeinflussenden Wirkstoffes mit mindestens einem Nitro-Statin liegt in der synergistischen antiproliferativen Wirkung, supplementiert durch antiinflammatorische, antithrombotische und vasodilatierende Wirkungen.

In einer besonders bevorzugten Ausführungsform besitzen das mindestens eine Nitro-Statin und der weitere pharmazeutische Wirkstoff eine unterschiedliche Freisetzungskinetik aus der Beschichtung des Implantats in das umliegende Implantationsgewebe. Beispielsweise können die unterschiedlichen Wirkstoffe in unterschiedlichen Schichten in der Beschichtung vorliegen, wobei die gegebenenfalls verwendete Trägermatrix des jeweiligen Wirkstoffs so gewählt ist, dass der weitere pharmazeutische Wirkstoff eine schnellere Freisetzung aus der Trägermatrix erfährt als das Nitro-Statin oder umgekehrt.

In einer weiteren besonderen Ausführungsform ist das erfindungsgemäße Implantat ein Stent und der weitere pharmazeutische Wirkstoff, bevorzugt Wirkstoffe aus der Klasse der antiproliferativen Mittel, werden mittels eines beschichteten Ballonkatheters an das Implantationsgewebe abgegeben. Zusätzlich kann der weitere pharmazeutische Wirkstoff in einer bioabbaubaren Trägermatrix, beispielsweise ein Hydrogel oder ein Polysaccharid, eingebettet sein, wobei die Trägermatrix beim Expandieren des Ballons an das Gewebe gedrückt wird und nach dem Deflatieren des Ballons am Gewebe haften bleibt. Auf diese Weise wird gewährleistet, dass der weitere pharmazeutische Wirkstoff steuerbar, mit einer bestimmten Konzentration freigesetzt wird.

In einem weiteren Aspekt betrifft die Erfindung die Verwendung mindestens eines Nitro-Statins in einer Beschichtung eines medizinischen Implantats, wobei die Beschichtung die Oberfläche des Implantats ganz oder in Teilen überzieht.

Die Verwendung mindestens eines Nitro-Statins in einer Beschichtung eines medizinischen Implantats hat den Vorteil, dass zum einen das umliegende Implantationsgewebe, welches mit der Beschichtung des Implantats in Kontakt steht, durch die Statin-Komponente dieses Wirkstoffs eine herausragende antithrombotische, antiinflammatorische und antiproliferative Wirkung erfährt und zum anderen durch die zusätzliche Freisetzung der NO-Komponente aus diesem Wirkstoff, einer Vasokonstriktion direkt vor Ort durch die Freisetzung des Wirkstoffs aus der Beschichtung des erfindungsgemäßen Implantats entgegengewirkt wird. Insbesondere wenn das medizinische Implantat aus einem biokorrodierbaren Werkstoff, wie Magnesium oder eine Legierung hiervon, besteht, können lokale Vasospasmen auftreten, welche von einer durch die Abbauprodukte des Werkstoffs bedingte Alkalose verursacht werden. Dies kann eine unerwünschte und verkürzte Standzeit des Implantats zur Folge haben. Durch die Verwendung mindestens eines Nitro-Statins in den erfindungsgemäßen Implantaten können Implantate hergestellt werden, die gegenüber den Auswirkungen einer Alkalose auf das Implantat unempfindlicher sind. Somit können längere Standzeiten der Implantate erreicht werden. Falls es sich beim erfindungsgemäßen Implantat um einen Stent handelt, kann die Gefahr einer Restenose bzw. der Beeinträchtigung des Gefäßlumens im und um den Stentbereich deutlich verringert werden. Somit kann der mit dem Einsatz des Implantats verfolgte Therapiezweck besser, über einen längeren Zeitraum und mit weniger Nebenwirkungen erreicht werden.

In einem weiteren Aspekt betrifft die Erfindung Nitro-Statine zur Prophylaxe oder Therapie einer Restenose oder einer Gefäßlumenbeeinträchtigung in einem Gefäßabschnitt, der mit einem Stent versorgt wurde, sowie die Verwendung von Nitro-Statinen zu einer solchen Prophylaxe oder Therapie.

Die Erfindung betrifft weiterhin Nitro-Statine zur Prophylaxe oder Therapie von lokalen Vasospasmen, sowie die Verwendung von Nitro-Statinen zu einer solchen Prophylaxe oder Therapie.

Nachfolgend wird die Erfindung anhand von Ausführungsbeispielen näher erläutert, welche den Erfindungsgegenstand aber nicht einschränken sollen.

### Ausführungsbeispiel 1: Beschichtung eines Stents mit einem in einer Trägermatrix enthaltenden Nitro-Statin

Als Trägermatrix wird PLGA verwendet. Dazu wird im ersten Schritt eine 4 %ige Lösung aus PLGA in Ethylacetat hergestellt (Verhältnis 65 % L-Laktid und 35 % Glykolid; die inhärente Viskosität dieses Polymers liegt bei 0,5 - 0,8 dL/g)

In 11,1 mL Ethylacetat Polymerlösung. Werden 0,53 mg NCX6560 solubilisiert. Die so hergestellte Lösung wird mit einem Tauchverfahren auf den Stent aufgebracht. Die Flächenbeladung von NCX6560 beträgt 0,75 µg/mm².

### Ausführungsbeispiel 2: Kavitätenfüllung eines Stents mit einem in einer Trägermatrix enthaltenden Nitro-Statin

Als Trägermatrix wird die in Ausfiihrungsbeispiel 1 hergestellte PLGA-Lösung verwendet

In dieser Lösung werden 0,60 mg Wirkstoff NCX6560 solubilisiert. Die so hergestellte Lösung wird mittels Mikroinjektion in die Kavitäten des Stents gebracht. Anschließend kann zusätzlich eine Beschichtung mindestens eines Nitro-Statins mit einem Tauchverfahren auf den Stent aufgebracht. Die Flächenbeladung von NCX6560 beträgt 0,5 - 1,6 µg/mm².

## Patentansprüche

1. Medizinisches Implantat, wobei die Oberfläche des Implantats ganz oder in Teilen mit einer Beschichtung, die aus mindestens einem Wirkstoff besteht oder mindestens einen Wirkstoff enthält, überzogen ist, **dadurch gekennzeichnet, dass** der Wirkstoff ein Nitro-Statin ist.

2. Implantat nach Anspruch 1, **dadurch gekennzeichnet, dass** das Nitro-Statin eine Verbindung der allgemeinen Formel (I) und ein pharmazeutisch annehmbares Salz oder Stereoisomer davon umfasst 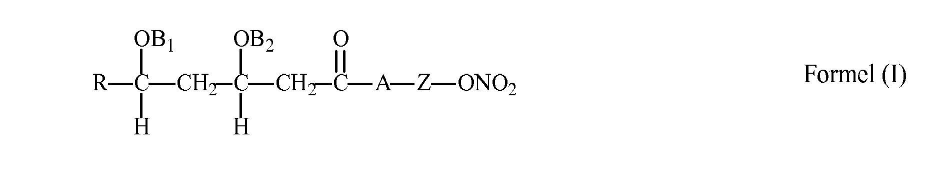 worin R 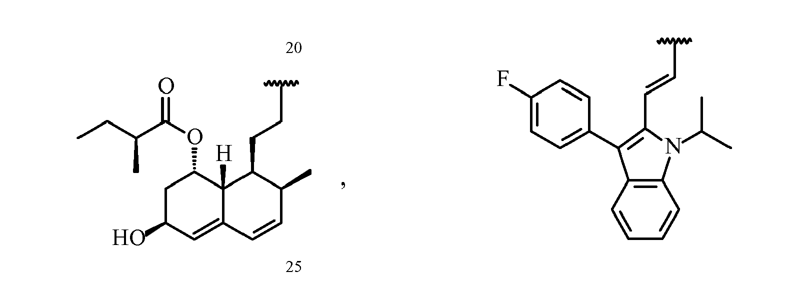 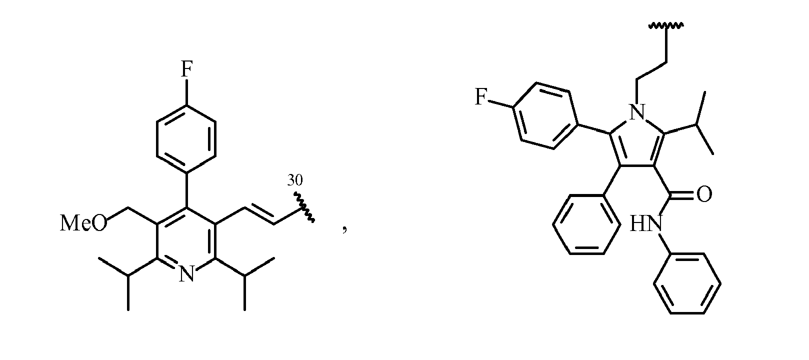 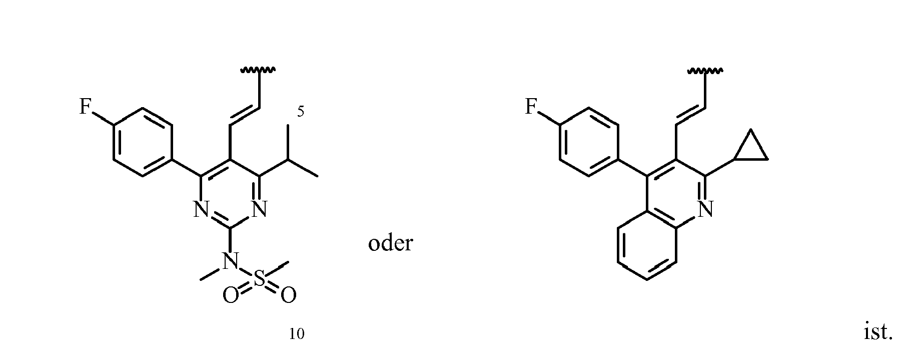 B₁ und B₂ gleich ist:
a) eine weitere NO-freisetzende Gruppe wie -NO₂, -ONO₂ oder Cinidomin (II)
b) eine Tosylat- oder Mesylat-Gruppe
c) ein geradkettiges oder verzweigtes C₁ bis C₂₀-Alkyl, bevorzugt C₁ bis C₁₀-Alkyl, das optional mit einem oder mehreren Substituenten substituiert ist, die aus der Gruppe ausgewählt werden, die aus folgenden bestehen: Halogenatomen, Hydroxy-,-ONO,
d) ein heterocyclischer gesättigter, ungesättigter oder aromatischer Ring, der ein oder mehrere Heteroatome enthält, die aus Stickstoff, Sauerstoff, Schwefel oder Halogenatomen ausgewählt werden, wobei der Ring zusätzlich mit Seitenketten substituiert ist, die ein geradkettiges oder verzweigtes Alkyl mit 1 bis 10 Kohlenstoffatomen aufweisen.
e) Cycloalkylen oder Cycloarylen mit 5 bis 7 Kohlenstoffatomen im Cycloalkylen-oder Cycloarylenring, wobei der Ring mit Seitenketten substituiert ist, die ein geradkettiges oder verzweigtes Alkyl mit 1 bis 10 Kohlenstoffatomen aufweisen, und optional ein oder mehrere Heteroatome enthält, die aus Stickstoff, Sauerstoff, Schwefel oder Halogenatomen ausgewählt werden,
wobei B₁ gleich oder verschieden B₂.
A gleich ein geradkettiges oder verzweigtes C₁ bis C₂₀-Alkylen, bevorzugt C₁ bis C₁₀-Alkyl, insbesondere -CH₂-; -O-, -S-, -NH- oder NR¹, worin R¹ ein geradkettiges oder verzeigtes C₁-C₁₀-Alkyl, bevorzugt CH₃, ist;
Z ein zweiwertiger Rest mit folgender Bedeutung ist:
a) geradkettiges oder verzweigtes C₁-C₂₀-Alkylen, bevorzugt C₁-C₁₀-Alkylen, das optional mit einem oder mehreren Substituenten substituiert ist, die aus der Gruppe ausgewählt werden, die aus folgenden bestehen: Halogenid, -OH, -OR¹, -COOH, - COOR¹, -NH2, NHR¹, NR¹R², worin R¹ und R² gleich oder verschieden sind und ein geradkettiges oder verzeigtes C₁-C₁₀-Alkyl sind;
b) Cycloalkylen mit 5 bis 7 Kohlenstoffatomen im Cycloalkylenring, worin der Cycloalkylenring optional mit einer oder mehreren geradkettigen oder verzeigten C₁-C₁₀-Alkyl-Seitenketten substituiert ist.
c) Arylalkylen, insbesondere 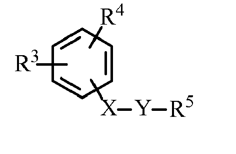 worin R³ gleich (CH₂)ₙ₁, worin n1 = 0 oder 20; R⁴ eine -COOH, -COOR¹, -OH, -OR¹ ist, worin R¹ wie oben definiert ist; X eine gesättigte oder ungesättigte -(CH₂)ₘ-Gruppe ist, worin m = 0-8; Y = -O-CO-, -COO-; R⁵ gleich (CH₂)ₙ₂, worin n2 = 1-20.
d) Heterocycloalkylen mit einem gesättigten, ungesättigten oder aromatischen 5- oder 6-gliedrigen Ring, der ein oder mehrere Heteroatome enthält, die aus Stickstoff, Sauerstoff, Schwefel ausgewählt werden, insbesondere folgende Gruppen: 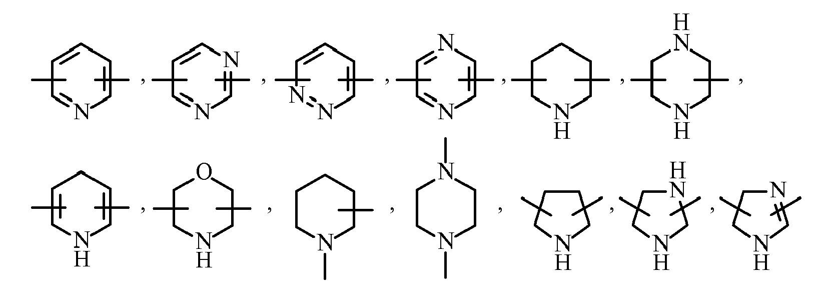
e) -O-, -Sᵢ-, -Se-, -NH- oder NR¹, worin i eine Zahl zwischen 1 bis 8 ist und R¹ ein geradkettiges oder verzeigtes C₁-C₁₀-Alkyl, bevorzugt CH₃, ist

3. Implantat nach einem der vorherigen Ansprüche, **dadurch gekennzeichnet, dass** das Implantat ein Stent ist.

4. Implantat nach Anspruch 3, **dadurch gekennzeichnet, dass** der Stent aus einem biokorrodierbaren metallischen Werkstoff besteht.

5. Implantat nach Anspruch 4, **dadurch gekennzeichnet, dass** der Stent aus einer Magnesiumlegierung besteht.

6. Implantat nach einem der vorherigen Ansprüche, **dadurch gekennzeichnet, dass** das mindestens eine Nitro-Statin in eine polymere Trägermatrix eingebettet ist.

7. Implantat nach einem der vorherigen Ansprüche, **dadurch gekennzeichnet, dass** das mindestens eine Nitro-Statin in einer Konzentration von 0,2 bis 3,5 µg/mm² Implantatoberfläche vorliegt.

8. Implantat nach einem der vorherigen Ansprüche, **dadurch gekennzeichnet, dass** die Beschichtung mindestens einen weiteren pharmazeutischen Wirkstoff ausgewählt aus der Gruppe bestehend aus antimikrobiellen, antimitotischen, antimyotischen, antineoplastischen, antiphlogistischen, antiproliferativen, antithrombischen und/oder vasodilatatorischenr Wirkstoffen enthält.

9. Verwendung von Nitro-Statinen zur Herstellung eines Implantats nach einem der Ansprüche 1 bis 8.

10. Nitro-Statine zur Prophylaxe oder Therapie von lokalen Vasospasmen.

11. Nitro-Statine zur Prophylaxe oder Therapie einer Restenose oder einer Gefäßlumenbeeinträchtigung in einem Gefäßabschnitt, der mit einem Stent versorgt wurde.
